# EUROPÄISCHE PATENTSCHRIFT

(11) **EP 0 943 138 B1**
(45) Veröffentlichungstag und Bekanntmachung des Hinweises auf die Patenterteilung: **22.05.2002**
(21) Anmeldenummer: 97954356.8
(22) Anmeldetag: 04.12.1997
(51) Int. Cl.: G09F 3/10, A61F 13/02, A61K 9/70

(54) **FLÄCHIGES SELBSTHAFTENDES WIRKSTOFFPFLASTER**
FLAT TRANSDERMAL MEDICATED SELF-ADHESIVE PATCH
TIMBRE TRANSDERMIQUE MEDICAMENTEUX AUTOCOLLANT MINCE

(30) Priorität: 04.12.1996 DE 19650329
(43) Veröffentlichungstag der Anmeldung: 22.09.1999
(73) Patentinhaber: Hexal AG, 83607 Holzkirchen (DE)
(72) Erfinder: KIBELE, Ralf, D-83607 Holzkirchen (DE); SCHLÜTER, Hans-Jürgen, D-83607 Holzkirchen (DE)
(74) Vertreter: Boeters, Hans Dietrich, Dr.
(86) Internationale Anmeldenummer: EP9706799
(87) Internationale Veröffentlichungsnummer: WO9825257

(56) Entgegenhaltungen:
- EP-A- 0 284 963
- EP-A- 0 418 607
- EP-A- 0 461 337
- WO-A-92/19451
- WO-A-95/00122
- DE-A- 3 823 070
- DE-A- 3 931 019

## Beschreibung

Abziehhilfen für Pflaster, transdermale therapeutische Systeme (TTS), Aufkleber oder Etiketten sind beispielsweise aus GB-A-2 179 910 und EP-B1-0 418 608 bekannt. Derartige Abziehhilfen werden durch teilweise geradlinige oder nichtgeradlinige Sollbruchstellen bzw. Schnitte in den Trägermaterialien (Abziehfolien) erzeugt und sollen das mechanische Ablösen der genannten Gegenstände vom Trägermaterial erleichtern.

Pflaster, beispielsweise transdermale therapeutische Systeme (TTS), werden in mehreren Schritten hergestellt vgl. beispielsweise WO 92/19451. Häufig ist es so, daß Wirk- und Hilfsstoffe direkt in einen Klebstoff eingearbeitet werden, so daß eine streichfähige wirkstoffhaltige Masse entsteht. Diese Masse wird großflächig auf eine Folienbahn für das Trägermaterial bzw. die Abziehfolie der Pflaster, aufgetragen, ggf. getrocknet und danach mit einer weiteren Folienbahn für die Abdeckfolie der Pflaster versehen. Erforderlichenfalls können derart erzeugte Verbunde für die weitere Verarbeitung in schmalere Bahnen geteilt werden. Danach werden Pflaster aus den Bahnen ausgestanzt, wobei neben den Pflastern in der Regel zwei Gitter entstehen.

So zeigt beispielsweise Figur 1 eine Draufsicht auf einen transparenten Verbund aus Abdeckfolie und Klebstoffschicht (Schraffur von links oben nach rechts unten) sowie Abziehfolie (Schraffur von rechts oben nach links unten). Wenn man sich die Folienbahn der Abziehfolie beim Blick auf die Zeichenebene als unterste Lage vorstellt, so sind die Sollbruchlinien von unten her eingestanzt worden. Nach Figur 2 sind zusätzlich bei Blick auf die Zeichenebene in die Folienbahn der Abdeckfolie mit Klebstoffschicht kreisförmige Schnitte eingestanzt, die in Längsrichtung der Bahn aufeinanderfolgende Pflaster definieren. Die Sollbruchlinien sind über die definierten Pflaster geführt, ohne daß die Sollbruchlinien miteinander verbunden sind bzw. ineinander übergehen. Danach kann man gemäß Figur 3b die Folienbahn aus dem Material der Abdeckfolie mit Klebstoffschicht abziehen, wobei die Folienbahn für das Trägermaterial bzw. die Abziehfolie mit einzelnen Pflastern gemäß Figur 3a zurückbleibt. Nach Figur 4a werden nun in der Folienbahn für das Trägermaterial (Abziehfolie) Schnitte vorgesehen, die das Trägermaterial bzw. die Abziehfolie der einzelnen Pflaster definieren, wonach die einzelnen Pflaster aus der Folienbahn herausgelöst werden, wobei einerseits die Folienbahn mit den ausgesparten bzw. entfernten Pflastern gemäß Figur 4b sowie Pflaster gemäß Figur 5 anfallen.

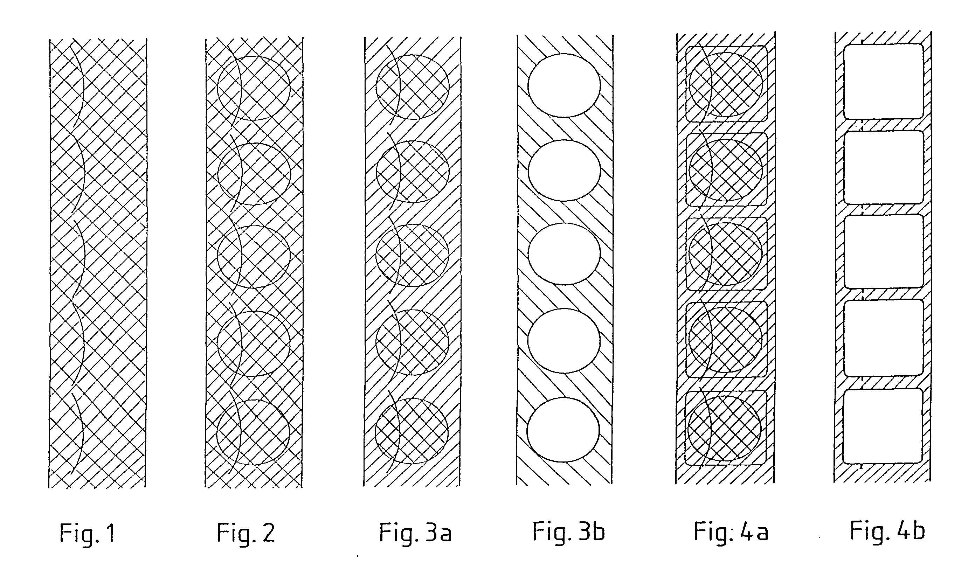

Beim Stanzen von Verbunden aus Abdeckfolie, Klebstoff und Abziehfolie kommt es mit den herkömmlichen Stanzformen bzw. Stanzmustern häufig zu Einrissen in den Stegen, beispielsweise in den Querstegen gemäß Figur 4b. Da hier die Endpunkte der Schnitte bzw. Sollbruchlinien aufeinander zu laufen, sind die Stege an diesen Stellen besonders gefährdet, zumal die Stege aus Gründen der Materialersparnis möglichst schmal gewählt werden.

Dasselbe Problem liegt vor, wenn man sich etwa zu dem in DE-C-3 931 019 Figur 1 gezeigten Pflaster die (nicht dargestellten) Stege vorstellt, in die die Sollbruchlinie 3 geführt ist, oder etwa zu dem in DE-C-3 344 334 Figur 1 dargestellten Pflaster analog die (nicht dargestellten) Stege hinzudenkt, in die die Sollbruchlinie 4 einschneidet, oder schließlich etwa zu dem in US-A-4 413 621 Figur 1 abgebildeten Pflaster die (nicht dargestellten) Stege ergänzt, in die die Sollbruchlinie 15 hineinschneidet.

Ferner beschreibt DE 38 23 070 mit Fig. 2, die dem gegenstand der Ansprüche 1 und 3 am nächsten koment ein flächiges selbsthaftendes Wirkstoffpflaster mit Abdeckfolie und selbstklebender Schicht, die wirkstoffhaltig ist, wobei das Pflaster zusätzlich mit einem entfernbaren Trägermaterial (Abziehfolie) versehen ist, wobei das Trägermaterial das Pflaster mit Abdeckfolie und selbstklebender Schicht mit einem Saum überragt und wobei eine Sollbruchlinie vorgesehen ist, die im Trägermaterial ausgebildet und über die selbstklebende Schicht geführt ist. Wenn man sich zu diesem bekannten Pflaster wiederum die (nicht dargestellten) Stege vorstellt, so darf man annehmen, daß die Sollbruchlinie in die Stege hineingeführt ist und damit auch in diesem Fall eine Situation geschaffen wird, die vorstehend diskutiert wurde.

Der Erfindung liegt nun die Aufgabe zugrunde, durch einen geeigneten Schnitt oder eine geeignete Sollbruchlinie ein Pflaster mit Abziehfolie zu schaffen, mit dem das geschilderte Einreißen der Stege vermieden werden kann und die Ausbeute an Pflastern erhöht wird.

Dazu wird erfindungsgemäß ein flächiges selbsthaftendes Wirkstoffpflaster mit Abdeckfolie und selbstklebender Schicht vorgesehen, die mindestens einen Wirkstoff enthält,
- wobei das Pflaster zusätzlich mit einem entfernbaren Trägermaterial (Abziehfolie) versehen ist,
- wobei das Trägermaterial das Pflaster mit Abdeckfolie und selbstklebender Schicht mit einem Saum überragt und
- wobei eine Sollbruchlinie im Trägermaterial ausgebildet und über die selbstklebende Schicht geführt ist, dadurch gekennzeichnet, daß der Saum mit zwei im Abstand voneinander angeordneten Aussparungen versehen ist, von denen jeweils einer der Endpunkte der Sollbruchlinie ausgeht.

Bei dem Pflaster kann es sich um ein TTS (transdermales therapeutisches system) handeln, bei dem die selbstklebende Schicht wirkstoffhaltig ist.

Zur Lösung der der Erfindung zugrundeliegenden Aufgabe wird ferner ein flächiges selbsthaftendes Wirkstoffpflaster mit Abdeckfolie, Reservoir, das mindestens einen Wirkstoff enthält, und Membran, die selbstklebend ist oder ein Klebemittel trägt, vorgesehen,
- wobei das Pflaster zusätzlich mit einem entfernbaren Trägermaterial (Abziehfolie) versehen ist,
- wobei das Trägermaterial das Pflaster mit Abdeckfolie sowie Reservoir und Membran mit einem Saum überragt und
- wobei eine Sollbruchlinie im Trägermaterial ausgebildet und über die selbstklebende Schicht geführt ist, dadurch gekennzeichnet, daß der Saum mit zwei im Abstand voneinander angeordneten Aussparungen versehen ist, von denen jeweils einer der Endpunkte Sollbruchlinie ausgeht,.

Die Aussparungen bzw. Einschnitte oder Einbuchtungen können etwa halbkreisförmig oder etwa V-förmig ausgebildet sein.

Das erfindungsgemäße Pflaster kann mehreckig, insbesondere rechteckig oder quadratisch, elliptisch oder kreisförmig ausgebildet sein und durch ein mehreckiges, insbesondere ein rechteckiges oder quadratisches Trägermaterial (Abziehfolie) abgedeckt sein. Selbstverständlich können die Ecken von mehreckigem Trägermaterial abgerundet sein.

Ferner kann das erfindungsgemäße Pflaster etwa kreisförmig ausgebildet sein und durch ein rundes, dreieckiges, hexagonales oder oktagonales Trägermaterial (Abziehfolie) abgedeckt sein.

Die Sollbruchlinien können als Einkerbung oder als Schnitt ausgebildet sein oder durch Perforationen gebildet werden.

Bei einer bevorzugten Ausführungsform des erfindungsgemäßen Pflasters überragt das Trägermaterial (Abziehfolie) das Pflaster allseitig.

Schließlich kann das erfindungsgemäße Pflaster mit Noppen in oder auf dem Trägermaterial versehen sein. Derartige Noppen können die Entnahme des Pflasters aus einer Verpackung erleichtern.

Eine weitere Ausführungsform der Erfindung betrifft ein Verfahren zur Herstellung von flächigen selbsthaftenden erfindungsgemäßen Wirkstoffpflastern (am nächsten kommender Stand der Technik ergibt sich aus WO 92/19451), bei dem man
(a) von einer Bahn eines mehrlagigen Verbunds ausgeht, der
   - eine Lage für die Abdeckfolie,
   - eine Lage für die selbstklebende Schicht oder das Reservoir mit Membran und
   - eine Lage für das entfernbare Trägermaterial (Abziehfolie) umfaßt, und
   in der Lage des entfernbaren Trägermaterials Sollbruchlinien vorsieht,
(b) gleichzeitig oder danach oder zuvor durch die Lage für die Abdeckfolie mit Lage für die selbstklebende Schicht bzw. das Reservoir mit Membran Schnitte führt, die jeweils ein Pflaster definieren,
(c) gleichzeitig oder danach oder zuvor durch die Lage für das entfernbare Trägermaterial (Abziehfolie) Schnitte führt, die jeweils das Trägermaterial (Abziehfolie) eines Pflasters definieren, und
(d) voneinander
   (i) eine Bahn eines Verbunds, der die Lage für die Abdeckfolie und die Lage für die selbstklebende Schicht bzw. das Reservoir mit Membran umfaßt, wobei die durch die Schnitte gemäß (b) definierten Pflaster ausgespart sind,
   (ii) eine Bahn des entfernbaren Trägermaterials, wobei die durch die Schnitte gemäß (c) definierten Trägermaterialien (Abziehfolien) jedes Pflasters ausgespart sind, und
   (iii) flächige selbsthaftende Wirkstoffpflaster mit entfernbarem Trägermaterial (Abziehfolie)
   separiert, dadurch gekennzeichnet, daß man in dem Saum, mit dem das Trägermaterial das Pflaster mit Abdeckfolie und selbstklebender Schicht überragt, zwei im Abstand voneinander angeordnete Aussparungen vorsieht, von denen jeweils einer der Endpunkte der Sollbruchlinie ausgeht.

Bei dem erfindungsgemäßen Verfahren kann man so vorgehen, daß man
- Maßnahme (a) durchführt,
- danach Maßnahme (b) durchführt,
- danach Bahn (d) (i) separiert,
- danach Maßnahme (c) durchführt und
- danach Bahn (d) (ii) und Pflaster (d) (iii) separiert.

Nachstehend wird die Erfindung anhand von Figuren noch näher erläutert. Es zeigen:
**Figuren 1 bis 4b** verschiedene Phasen bei der Herstellung bekannter Pflaster;

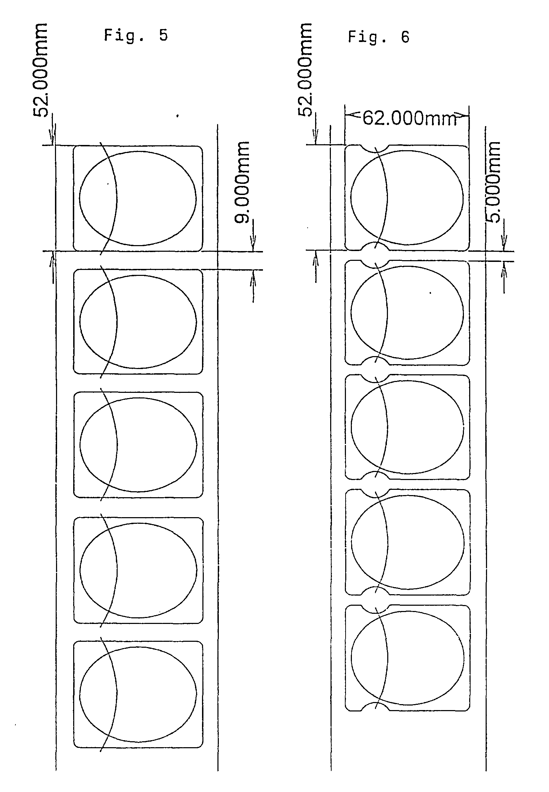

**Figur 5** eine zu **Figur 4a** analoge Bahn für ein anderes bekanntes Pflaster;
**Figur 6** eine zu den **Figuren 4a** und **5** analoge Bahn für die Herstellung erfindungsgemäßer Pflaster;

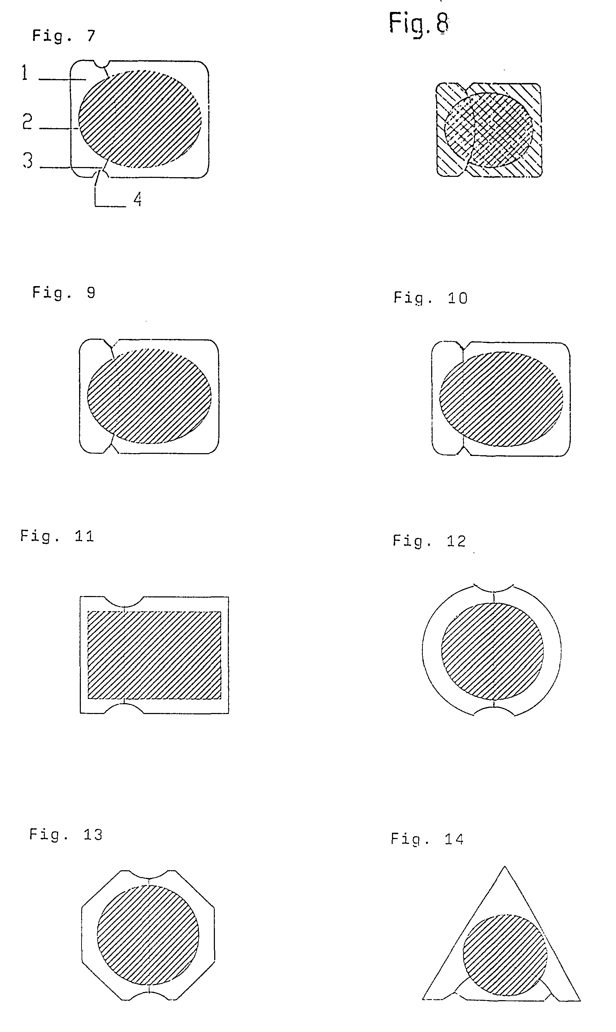

**Figuren 7** bis **14** erfindungsgemäße Pflaster jeweils mit Abziehfolie.

Mit den nachfolgend beschriebenen Stanzformen (**Fig. 6** bis **14**) konnte sowohl der Materialverbrauch verringert als auch die zeitliche Nutzung der Stanzen durch eine erhöhte Betriebssicherheit (deutlich weniger Bahnrisse) erheblich erhöht werden. Beispielsweise wurde bei **Fig. 6** bis **8** der Zwischenraum auf 40 bis 60 % der ursprünglichen Breite (**Fig. 1** bis **5**) reduziert.

Für das Pflaster von Fig. 7 wurde gefunden, daß durch die erfindungsgemäßen Aussparungen die für die Produktion von Pflastern erforderliche Gesamtmenge an Laminat um 5 bis 10 % reduziert werden kann. Dies wird dadurch möglich, daß die zur Trennung der mit Abziehfolie versehenen Pflaster erforderlichen Stege bzw. Zwischenräume verringert werden, was zu einer beachtlichen Einsparung an Material führt. Dies wird im folgenden anhand des Beispiels eines 20 cm²-Pflasters erläutert. Bei konventionellen Stanzformen mußte ein Zwischenraum von mindestens 9 mm verbleiben (**Figur 5**), um eine sichere Verarbeitung zu gewährleisten. Bei den erfindungsgemäßen Stanzformen konnte dieser Zwischenraum auf bis zu 4 mm reduziert werden, und zwar bei gleicher oder besserer Verarbeitungsfähigkeit. Eine Reduzierung des Zwischenraumes zwischen zwei Pflastern à 20 cm² um 5 mm führt zu einer Materialersparnis von 8 %.

Der Vorteil der besseren zeitlichen Nutzung der Stanzen ergibt sich daraus, daß jeder Bahnriß zu Verklebungen in der Stanze und damit zu aufwendigen Reinigungsarbeiten führt und, falls in einer Linie weitere Schritte wie Bedrucken oder Verpacken der Pflaster durchgeführt werden, Folgeausfälle möglich, wenn nicht sogar zu erwarten sind (z. B. Verschmutzung von Siegelbacken bei heißsiegelfähiger Verpackungsfolie durch Zerfließen der siegelfähigen Schicht). Üblicherweise kann man bei jedem Bahnriß erfahrungsgemäß mit einer Stillstandszeit von 15 bis 30 Minuten rechnen. Bei handelsüblichen Hubstanzen beispielsweise sind Taktraten von 100 bis 120 pro Minute üblich. Somit läßt sich für jede Stillstandszeit eine Mindererzeugung von 1500 bis 3600 Pflastern pro Bahn ansetzen.

Die Form der erfindungsgemäßen Aussparungen kann symmetrisch oder unsymmetrisch bogenförmig oder dreieckartig sein, wobei es bei einer gerundeten bogenförmigen Aussparung nicht darauf ankommt, daß das Ende der Sollbruchstelle bzw. des Schnitts direkt in der Mitte des Bogens liegt. Bei einer Ausführungsform mit einer dreieckartigen Aussparung sollte das Ende der Sollbruchstelle bzw. des Schnitts allerdings ziemlich genau in der Spitze des Dreiecks liegen.

Die erfindungsgemäßen Aussparungen lassen sich auf jede Art von flexiblen Abziehfolien anwenden. Für transdermale Systeme geeignete Abziehfolien, wie z. B. Polyethylenterephthalat-, Polyethylen-, Polypropylen- oder Polyvinylchlorid-Folien oder Verbundfolien (u. a. Papier/Polyethylen), gehören zum Stand der Technik und können diesem entnommen werden. Die Abziehfolie kann mit einer Trennmittelbeschichtung versehen sein, sie kann beispielsweise silikonisiert oder fluorosilikonisiert sein.

Selbstverständlich lassen sich die erfindungsgemäßen Aussparungen auf bogenförmige, nicht oder teilweise geradlinige und geradlinige Sollbruchstellen oder Schnitte anwenden. Auch sind die Aussparungen nicht auf bestimmte Größen von Abziehfolien beschränkt. Handelsübliche Größen von transdermalen therapeutischen Systemen liegen beispielsweise zwischen 5 cm² und 100 cm², wobei die wirksame Fläche rund, oval oder vieleckig (ggf. abgerundet) sein kann.

**Figuren 7** und **8** zeigen erfindungsgemäße Pflaster, die sich von einem bekannten Pflaster gemäß **Figuren 1** bis **5** durch zwei sich gegenüberliegende Aussparungen im Saum der Abziehfolie unterscheiden, wobei die Sollbruchlinie in diese Aussparungen einmündet.

**Figur 7** zeigt eine rechteckige Trägerschicht bzw. Abziehfolie 1, auf der das Pflaster 2 mittig angeordnet ist. Das Pflaster 2 ist vorzugsweise ein auf der Abziehfolie haftendes bzw. klebriges transdermales therapeutisches System. Der Schnitt bzw. die Sollbruchstelle 3 enden an runden Aussparungen 4.

**Figuren 8** bis **14** zeigen weitere Ausführungsformen der erfindungsgemäßen Pflaster.

## Patentansprüche

1. Flächiges selbsthaftendes Wirkstoffpflaster (2) mit Abdeckfolie und selbstklebender Schicht, die mindestens einen Wirkstoff enthält
- wobei das Pflaster (2) zusätzlich mit einem entfernbaren Trägermaterial (Abziehfolie) (1) versehen ist,
- wobei das Trägermaterial (1) das Pflaster (2) mit Abdeckfolie und selbstklebender Schicht mit einem Saum überragt und
- wobei eine Sollbruchlinie (3) im Trägermaterial (1) ausgebildet und über die selbstklebende Schicht geführt ist, **dadurch gekennzeichnet, daß** der Saum mit zwei im Abstand voneinander angeordneten Aussparungen (4) versehen ist, von denen jeweils einer der Endpunkte der Sollbruchlinie (3) ausgeht.

2. Pflaster nach Anspruch 1, **dadurch gekennzeichnet, daß** es sich bei dem Pflaster um ein transdermales therapeutisches System (TTS) handelt, bei dem die selbstklebende Schicht wirkstoffhaltig ist.

3. Flächiges selbstklebendes Wirkstoffpflaster (2) mit Abdeckfolie, Reservoir, das mindestens einen Wirkstoff enthält, und Membran, die selbstklebend ist oder ein Klebemittel trägt,
- wobei das Pflaster (2) zusätzlich mit einem entfernbaren Trägermaterial (Abziehfolie) (1) versehen ist,
- wobei das Trägermaterial (1) das Pflaster (2) mit Abdeckfolie sowie Reservoir und Membran mit einem Saum überragt und
- wobei eine Sollbruchlinie (3) im Trägermaterial (1) ausgebildet und über die selbstklebende Schicht geführt ist, **dadurch gekennzeichnet, daß** der Saum mit zwei im Abstand voneinander angeordneten Aussparungen (4) versehen ist, von denen jeweils einer der Endpunkte der Sollbruchlinie (3) ausgeht.

4. Pflaster nach einem der vorhergehenden Ansprüche, **dadurch gekennzeichnet, daß** die Aussparungen etwa halbkreisförmig oder etwa V-förmig ausgebildet sind.

5. Pflaster nach einem der vorhergehenden Ansprüche, **dadurch gekennzeichnet, daß** es mehreckig, insbesondere rechteckig oder quadratisch, elliptisch oder kreisförmig ausgebildet ist und durch ein mehreckiges, insbesondere rechteckiges oderquadratisches Trägermaterial (Abziehfolie) abgedeckt ist.

6. Pflaster nach einem der Ansprüche 1 bis 4, **dadurch gekennzeichnet, daß** es etwa kreisförmig ausgebildet ist und durch ein rundes, dreieckiges, hexagonales oder oktagonales Trägermaterial (Abziehfolie) abgedeckt ist.

7. Pflaster nach einem der vorhergehenden Ansprüche, **dadurch gekennzeichnet, daß** die Sollbruchlinie als Einkerbung oder als Schnitt ausgebildet ist oder durch Perforationen gebildet wird.

8. Pflaster nach einem der vorhergehenden Ansprüche, **dadurch gekennzeichnet, daß** das Trägermaterial (Abziehfolie) das Pflaster allseitig überragt.

9. Pflaster nach einem der vorhergehenden Ansprüche, **dadurch gekennzeichnet, daß** es mit Noppen in bzw. auf dem Trägermaterial versehen ist.

10. Verfahren zur Herstellung von flächigen selbsthaftenden Wirkstoffpflastern (Fig. 7: 2), bei dem man
(a) von einer Bahn eines mehrlagigen Verbunds ausgeht, der
- eine Lage für die Abdeckfolie,
- eine Lage für die selbstklebende Schicht oder das Reservoir mit Membran und
- eine Lage für das entfernbare Trägermaterial (Abziehfolie) umfaßt, und
in der Lage des entfernbaren Trägermaterials Sollbruchlinien vorsieht (Fig. 1),
(b) gleichzeitig oder danach oder zuvor durch die Lage für die Abdeckfolie mit Lage für die selbstklebende Schicht bzw. das Reservoir mit Membran Schnitte führt, die jeweils ein Pflaster definieren (Fig. 2),
(c) gleichzeitig oder danach oder zuvor durch die Lage für das entfernbare Trägermaterial (Abziehfolie) Schnitte führt, die jeweils das Trägermaterial (Abziehfolie) eines Pflasters definieren (Fig. 4a), und
(d) voneinander separiert:
(i) eine Bahn eines Verbunds, der die Lage für die Abdeckfolie und die Lage für die selbstklebende Schicht bzw. das Reservoir mit Membran umfaßt (Fig. 3b), wobei die durch die Schnitte gemäß (b) definierten Pflaster ausgespart sind,
(ii) eine Bahn des entfernbaren Trägermaterials (Fig. 4b), wobei die durch die Schnitte gemäß (c) definierten Trägermaterialien (Abziehfolien) jedes Pflasters ausgespart sind, und
(iii) flächige selbsthaftende Wirkstoffpflaster (Fig. 7: 2) mit entfernbarem Trägermaterial (Abziehfolie) (Fig. 7: 1), **dadurch gekennzeichnet, daß** man in dem Saum, mit dem das Trägermaterial (Fig. 7: 1) das Pflaster (Fig. 7: 2) mit Abdeckfolie und selbstklebender Schicht überragt, zwei im Abstand voneinander angeordnete Aussparungen (Fig. 7: 4) vorsieht, von denen jeweils einer der Endpunkte der Sollbruchlinie (Fig. 1; Fig. 7: 3) ausgeht.

11. Verfahren nach Anspruch 10, **dadurch gekennzeichnet, daß** man
- Maßnahme (a) durchführt,
- danach Maßnahme (b) durchführt,
- danach Bahn (d) (i) separiert,
- danach Maßnahme (c) durchführt und
- danach Bahn (d) (ii) und Pflaster (d) (iii) separiert.

## Claims

1. A flat self-adhering drug patch (2) comprising a cover foil and self-adhesive layer containing at least one active agent,
- where the patch (2) additionally comprises a removable carrier material (peeling foil) (1) and
- where the carrier material (1) extends beyond the patch (2) with cover foil and self-adhesive layer to form a border, and
- where a break line (3) is formed in the carrier material (1) and passes over the self-adhesive layer, **characterized in that** the border is provided with two recesses (4) spaced apart from one another, and wherein the respective end points of the break line (3) extend from the recesses (4).

2. Patch according to claim 1, **characterized in that** the patch is a transdermal therapeutic system (TTS) wherein the self-adhesive layer is drug-containing.

3. A flat self-adhering drug patch (2) with a cover layer, reservoir, containing at least one active agent and a membrane which is self-adhesive or carries an adhesive,
- wherein the patch (2) additionally comprises a removable carrier material (peeling foil) (1) and
- wherein the carrier material (1) extends beyond the patch (2) with its cover foil as well as reservoir and membrane to form a border, and
- where a break line (3) is formed in the carrier material (1) and passes over the self-adhesive layer, **characterized in that** the border is provided with two recesses (4) spaced from one another and wherein the end points of the break line (3) extend from the recesses (4).

4. Patch according to any one of the preceding claims, **characterized in that** the recesses are approximately semicircular or approximately V-shaped.

5. Patch according to any one of the preceding claims, **characterized in that** the patch is multi-cornered, in particular rectangular or square, elliptic or circular and covered with a multi-cornered, in particular rectangular or square carrier material (peeling foil) .

6. Patch according to any one of the claims 1 to 4, **characterized in that** the patch is approximately circular and covered with a round, triangular, hexagonal or octagonal carrier material (peeling foil) .

7. Patch according to any one of the preceding claims, **characterized in that** the break line is formed as an indentation or as a cut or is formed by perforations.

8. Patch according to any one of the preceding claims, **characterized in that** the carrier material (peeling foil) extends beyond all sides of the patch.

9. Patch according to any one of the preceding claims, **characterized in that** bumps are provided in or on the carrier material.

10. Method for manufacturing flat self-adhering drug patches (fig. 7 : 2) wherein
(a) a band of a multi-layer composite is provided comprising
- a layer for the cover foil,
- a layer for the self-adhesive layer or the reservoir with membrane, and
- a layer for the removable carrier material (peeling foil), and
providing break lines in the removable carrier material (fig. 1),
(b) cuts are made through the layer for the cover foil with the self-adhesive layer or with the reservoir and membrane to define the patches, the cuts made simultaneously or after or before (fig. 2),
(c) cuts are made in the layer for the removable carrier material (peeling foil), thereby defining the carrier material (peeling foil) of the patches, the cuts being made simultaneously or after or before (fig. 4a), and
(d)
(i) a band of the composite comprising the layer for the cover foil and the self-adhesive layer or the reservoir with membrane (fig. 3b), whereby the patches are defined by the cuts in step (b),
(ii) a band of the removable carrier material (peeling foil) (fig. 4b), whereby the carrier material (peeling foil) of each patch defined by the cuts according to step (c), and
(iii) flat self-adhering drug patches with removable carrier material (peeling foil) (fig. 7 : 1),
are separated from each other, **characterized in that** the border by which the carrier material (fig. 7: 1) extend beyond the patch (fig. 7: 2) with cover foil and self-adhesive layer, is provided with two recesses (fig. 7: 4) spaced apart from one another, and wherein the respective end points of the break line (fig. 1; fig. 7: 3) extend from the recesses.

11. Method according to claim 10, **characterized in that**
- step (a) is performed,
- thereafter step (b) is performed,
- thereafter the band (d) (i) is separated,
- thereafter step (c) is performed and
- thereafter the band (d) (ii) and the patches (d) (iii) are separated.

## Revendications

1. Pansement auto-adhésif plat de substance active (2) avec feuille de recouvrement et film auto-adhésif, qui contient au moins une substance active, dans lequel
- le pansement adhésif (2) comporte, en outre, une matière support détachable (feuille pelable) (1),
- la matière support (1) a une bordure qui dépasse du pansement adhésif (2) avec feuille de recouvrement et film auto-adhésif, et
- une ligne destinée à la rupture (3) est formée dans la matière support (1) et s'étend en travers du film auto-adhésif, **caractérisé en ce que** la bordure comporte deux encoches (4) éloignées l'une de l'autre à partir desquelles part chacune des extrémités de la ligne destinée à la rupture (3).

2. Pansement adhésif selon la revendication 1, **caractérisé en ce que** le pansement adhésif est un système thérapeutique transdermique (TTS) dans lequel le film auto-adhésif contient au moins une substance active.

3. Pansement auto-adhésif plat de substance active (2) avec feuille de recouvrement, réservoir qui contient au moins une substance active, et membrane qui est auto-adhésive ou qui porte un adhésif, dans lequel
- le pansement adhésif (2) comporte, en outre, une matière support pelable (feuille pelable) (1),
- la matière support (1) a une bordure qui dépasse du pansement adhésif (2) avec feuille de recouvrement ainsi que réservoir et membrane, et
- une ligne destinée à la rupture (3) est formée dans la matière support (1) et s'étend en travers du film auto-adhésif, **caractérisé en ce que** la bordure comporte deux encoches (4) éloignées l'une de l'autre à partir desquelles part chacune des extrémités de la ligne destinée à la rupture (3).

4. Pansement auto-adhésif selon l'une des revendications précédentes, **caractérisé en ce que** les encoches ont une configuration à peu près semi-circulaire ou à peu près en V.

5. Pansement auto-adhésif selon l'une des revendications précédentes, **caractérisé en ce qu'**il a une configuration polygonale, en particulier rectangulaire ou carrée, elliptique ou circulaire et qu'il est recouvert d'une matière support (feuille pelable) polygonale, en particulier rectangulaire ou carrée.

6. Pansement auto-adhésif selon l'une des revendications 1 à 4, **caractérisé en ce qu'**il a une configuration à peu près circulaire et qu'il est recouvert d'une matière support (feuille pelable) ronde, triangulaire, hexagonale ou octogonale.

7. Pansement auto-adhésif selon l'une des revendications précédentes, **caractérisé en ce que** la ligne destinée à la rupture est configurée comme une rainure ou comme une entaille ou est formée par des perforations.

8. Pansement auto-adhésif selon l'une des revendications précédentes, **caractérisé en ce que** la matière support (feuille pelable) dépasse de tous les côtés du pansement adhésif.

9. Pansement auto-adhésif selon l'une des revendications précédentes, **caractérisé en ce qu'**il comporte des nopes dans ou sur la matière support.

10. Procédé pour la fabrication de pansements auto-adhésifs plats de substance active (figure 7 : 2), dans lequel
(a) on part d'une bande d'un composé multicouche qui comprend
- une couche pour la feuille pelable,
- une couche pour le film auto-adhésif ou le réservoir avec membrane, et
- une couche pour la matière support détachable (feuille pelable), et
on prévoit des lignes destinées à la rupture dans la couche de la matière support détachable (figure 1),
(b) simultanément ou après ou avant, on pratique à travers la couche de la feuille de recouvrement avec la couche pour le film auto-adhésif ou le réservoir avec membrane des entailles qui définissent chacune un pansement auto-adhésif (figure 2),
(c) simultanément ou après ou avant, on pratique à travers la couche de la matière support détachable (feuille pelable) des entailles qui définissent chacune la matière support (feuille pelable) d'un pansement auto-adhésif (figure 4a), et
(d) on sépare les uns des autres :
(i) une bande d'un composé qui comprend la couche pour la feuille de recouvrement et la couche pour le film auto-adhésif ou le réservoir avec membrane (figure 3b), les pansements adhésifs définis par les entailles suivant (b) étant ainsi évidés,
(ii) une bande de la matière support détachable (figure 4b), les matières supports (feuilles pelables) de chaque pansement adhésif définies par les entailles suivant (c) étant ainsi évidées, et
(iii) des pansements plats auto-adhésifs de substance active (figure 7 : 2) avec matière support détachable (feuille pelable) (figure 7 : 1), **caractérisé en ce que** l'on ménage, dans la bordure avec laquelle la matière support (figure 7 : 1) dépasse du pansement adhésif (figure 7 : 2) avec feuille de recouvrement et film auto-adhésif, deux encoches (figure 7 : 4) éloignées l'une de l'autre à partir desquelles part chacune des extrémités de la ligne destinée à la rupture (figure 1 ; figure 7 : 3).

11. Procédé selon la revendication 10, **caractérisé en ce que**
- l'on procède à l'étape (a),
- ensuite on procède à l'étape (b),
- ensuite on sépare la bande (d) (i),
- ensuite on procède à l'étape (c), et
- ensuite on sépare la bande (d) (ii) et le pansement adhésif (d) (iii).
